(19) Europäisches Patentamt
European Patent Office
Office européen des brevets

(11) Veröffentlichungsnummer: **0 292 824**
**B1**

(12) # EUROPÄISCHE PATENTSCHRIFT

(45) Veröffentlichungstag der Patentschrift: **01.08.90**

(51) Int. Cl.[5]: **C07C 17/12**

(21) Anmeldenummer: **88107804.2**

(22) Anmeldetag: **16.05.88**

(54) **Verfahren zur Kernchlorierung von aromatischen Kohlenwasserstoffen.**

(30) Priorität: **28.05.87 DE 3718060**

(43) Veröffentlichungstag der Anmeldung:
**30.11.88 Patentblatt 88/48**

(45) Bekanntmachung des Hinweises auf die Patenterteilung:
**01.08.90 Patentblatt 90/31**

(84) Benannte Vertragsstaaten:
**BE CH DE FR GB IT LI**

(56) Entgegenhaltungen:
**EP-A- 0 126 669**

(73) Patentinhaber: **BAYER AG,**
**D-5090 Leverkusen 1 Bayerwerk(DE)**

(72) Erfinder: **Mais, Franz-Josef, Dr., Kirchfeldstrasse 69,**
**D-4000 Düsseldorf 1(DE)**
Erfinder: **Fiege, Helmut, Dr., Walter-Flex-Strasse 23,**
**D-5090 Leverkusen 1(DE)**
Erfinder: **Röhlk, Kai, Dr., Odenthaler Markweg 36,**
**D-5060 Bergisch Gladbach 2(DE)**
Erfinder: **Wedemeyer, Karlfried, Dr., Bilharzstrasse 7,**
**D-5000 Köln 80(DE)**

EP 0 292 824 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Kernchlorierung von aromatischen Kohlenwasserstoffen in Gegenwart von Friedel-Crafts-Katalysatoren und in Gegenwart von Co-Katalysatoren in flüssiger Phase.

Die Umsetzung von aromatischen Kohlenwasserstoffen, wie Toluol, in flüssiger Phase mit gasförmigem Chlor zu kernsubstituierten Chlorderivaten, wie Monochlortoluol, ist aus der Literatur bekannt (Ullmanns Encyklopädie der technischen Chemie, 4. Auflage, Band 9, Seite 499 ff). Man führt die Chlorierung im allgemeinen in Gegenwart von Friedel-Crafts-Katalysatoren, wie Eisen(III)-chlorid, Antimonchloriden oder Aluminiumchlorid, durch. Das erhaltene Chlorierungsprodukt ist gewöhnlich eine Mischung aus stellungsisomeren monochlorierten und polychlorierten Verbindungen. So erhält man beispielsweise bei der mit Eisen(III)-chlorid katalysierten Chlorierung von Toluol eine Mischung aus Monochlortoluolen und Dichlortoluolen. In der Monochlortoluolfraktion ist das Hauptprodukt o-Chlortoluol. Daneben ist in ihr p-Chlortoluol enthalten sowie ein geringer Anteil an m-Chlortoluol.

Da besonders p-Chloralkylbenzole, wie p-Chlortoluol, wertvolle Zwischenprodukte darstellen, hat es in der Vergangenheit nicht an Versuchen gefehlt, die Chlorierung so zu lenken, daß das Verhältnis von 0- zu p-Chloralkylbenzolen erniedrigt wird, d.h. man versuchte, Bedingungen zu finden, die die Bildung von p-Chloralkylbenzolen begünstigten.

Aus der US-Patentschrift 3 226 447 ist bekannt, daß durch Zusatz von Schwefelverbindungen mit divalentem Schwefel zum Friedel-Crafts-Katalysator bei der Chlorierung von z.B. Toluol ein o/p-Verhältnis von 1,2 erhalten werden kann. Nachteilig bei diesem Verfahren ist die Tatsache, daß man dieses günstige Verhältnis nur bei Anwendung von Antimonsalzen als Friedel-Crafts-Katalysatoren erreicht. Nachteilig ist weiterhin, daß die Aufwandmengen der Katalysatorkomponenten gemäß dem Beispiel 16 sehr hoch liegen, nämlich bei 1 Gew.-% für jede der beiden Komponenten. Nachteilig ist ferner, daß das o/p-Verhältnis noch deutlich größer als 1 ist, also mehr o-als p-Verbindung entsteht.

In der DE-OS 1 543 020 und US 4 031 144 wird die Chlorierung von Toluol mit Friedel-Crafts-Katalysatoren oder Verbindungen, die Friedel-Crafts-Katalysatoren unter den Reaktionsbedingungen bilden, bei Zusatz von Schwefel oder Schwefelverbindungen, wie Schwefelhalogeniden, beschrieben. Man erhält dabei ein Verhältnis für die Toluolchlorierung von o/p = 1,03 - 1,10 (siehe Beispieltabelle der US 4 031 144). Die Aufwandmengen liegen z.B. gemäß Beispiel 9 der DE-OS 1 543 020 bei 0,05 Gew.-% $S_2Cl_2$ und 0,10 Gew.-% $FeCl_3$. Nachteilig ist bei diesem Verfahren, daß das o/p-Verhältnis noch unbefriedigend hoch ist.

Aus US 4 031 147, US 4 069 263, US 4 069 264 und US 4 250 122 ist die Chlorierung von Toluol mit Friedel-Crafts-Katalysatoren unter Zusatz von Thianthrenen oder substituierten Thianthrenen bekannt. Die für die Chlorierung von z.B. Toluol erreichbaren günstigsten o/p-Verhältnisse liegen bei ca. 0,7. Nachteilig bei diesem Verfahren ist jedoch, daß dieses günstige Verhältnis entweder nur durch die Verwendung von Antimonsalzen als Friedel-Crafts-Katalysator oder im Falle der Verwendung von Eisensalzen als Katalysatoren nur bei sehr niedrigen Reaktionstemperaturen von etwa 0°C (siehe Beispiel 14 der US 4 250 122 und Beispiele 2-4 der US 4 069 263) erreicht wird. Beides ist technisch ausgesprochen ungünstig. Die co-katalytische Wirkung der Thianthrene wird beim Einsatz von Antimonsalzen durch in der Technik nur außerordentlich aufwendig zu vermeidende Eisenspuren stark behindert (siehe dazu US 4 024 198). Zudem ist die Reaktion so stark exotherm, daß eine Abführung der Wärme bei ca. 0°C sehr aufwendig (Solekühlung) ist. Weiterhin ist nachteilig, daß - wie die DE-OS 3 023 437 lehrt -die Thianthrene unter üblichen Reaktionsbedingungen bereits von allgegenwärtigen Wasserspuren zerstört werden und somit ihre Wirksamkeit verlieren.

Aus den Patentschriften US 4 289 916, EP 0 063 394 und EP 0 173 222 ist die Chlorierung von Toluol in Gegenwart von Lewis-Säuren und Phenoxathiinen als Katalysatorsystem bekannt. Das erreichbare o/p-Verhältnis beträgt in einem Fall 0,6 (siehe Beispiel 1 der EP 0 173 222). Nachteilig ist daran jedoch die technisch äußerst ungünstige Verwendung von Antimonchlorid und die hohe Aufwandmenge von 0,29 Gew.-% an Co-Katalysator (siehe Beispiel 1 der EP 0 173 222). Bei Verwendung von Eisen(III)-chlorid anstatt Antimonchlorid ergibt sich ein o/p-Verhältnis von 0,68 (siehe Beispiel 3 der EP 0 173 222), dies jedoch wiederum nur bei der technisch äußerst ungünstigen niedrigen Reaktionstemperatur von 5°C. Bei der technisch vorteilhaften Reaktionstemperatur von 50°C ist das o/p-Verhältnis mit dem in EP 0 173 222 beanspruchten Phenoxathiinderivat in Gegenwart von Eisen(III)-chlorid nur noch 0,88, wie von uns durchgeführte Versuche zeigen (siehe Beispiel 40). In den anderen beiden Patentveröffentlichungen (US 4 289 916 und EP 0 063 384) sind die besten Beispiel mit Aufwandmengen von je 0,05 Gew.-% Eisen(III)-chlorid und 0,05 Gew.-% eines Phenoxathiinderivats bei 35°C Reaktionstemperatur und einem günstigsten o/p-Verhältnis von ca. 0,8 beschrieben. Verwendet man hier anstelle von Eisen(III)-chlorid die technisch ungünstigeren Antimonchloride, so kann das o/p-Verhältnis bei 20°C Reaktionstemperatur bis auf 0,65 gesenkt werden (siehe Beispiel Nr. 16 der EP 0 063 384). Nachteilig ist jedoch, daß auch Phenoxathiine in Gegenwart von Wasserspuren zerstört werden.

Aus der Patentschrift EP 0 126 669 ist die Chlorierung von Toluol in Gegenwart von Friedel-Crafts-Katalysatoren und N-substituierten Phenothiazinen bekannt. Gemäß dem günstigsten Beispiel (siehe Beispiel Nr.1 der EP 0 126 669) ergibt sich bei 30°C Reaktionstemperatur und Einsatzmengen von 0,011

Gew.-% Eisen(III)-chlorid und 0,028 Gew.-% Co-Katalysator ein o/p-Verhältnis von 0,84. Das o/p-Isomerenverhältnis ist bei diesem Verfahren noch ungünstig hoch.

Aus EP 0 112 722 und EP 0 154 236 ist die Chlorierung von beispielsweise Toluol in Gegenwart von bestimmten Zeolithen bekannt. Das o/p-Verhältnis liegt bei Zusatz von beispielsweise Halogencarbonsäurehalogeniden als Moderatoren bei ca. 0,3. Nachteilig an diesem Verfahren sind die erheblichen Aufwandmengen von 5 Gew.-% Zeolith und 1 Gew.-% Halogencarbonsäurehalogeniden. Weiterhin ist von erheblichen Nachteil, daß in den erhältlichen Gemischen sehr große Mengen (bis zu 8 Gew.-%) an Benzylchloriden erhalten werden, wie eigene Versuche zeigten. Die Bildung von Benzylchloriden stört die nachfolgende übliche destillative Aufarbeitung in ganz außerordentlichem Maße.

Es wurde nun ein Verfahren zur Kernchlorierung von aromatischen Kohlenwasserstoffen der Formel

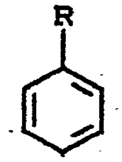

(I)

worin R einen Alkyl- oder Cycloalkylrest mit bis zu 12 C-Atomen bedeutet,
in Gegenwart von Friedel-Crafts-Katalysatoren und in Gegenwart von Co-Katalysatoren in flüssiger Phase gefunden, das dadurch gekennzeichnet ist, daß man als Co-Katalysatoren Thiazepine einsetzt.

Als Thiazepine können solche der unten angeführten Formeln in das erfindungsgemäße Verfahren eingesetzt werden:

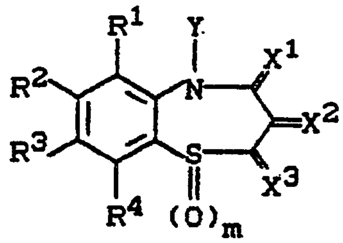

(II)

oder

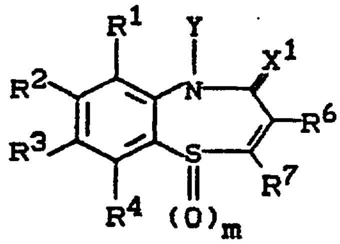

(III)

oder

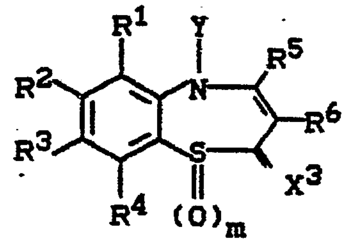

(IV)

oder

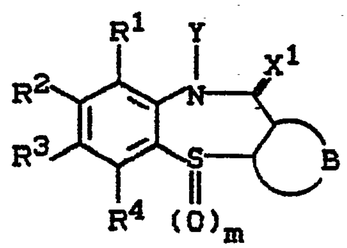

(V)

oder

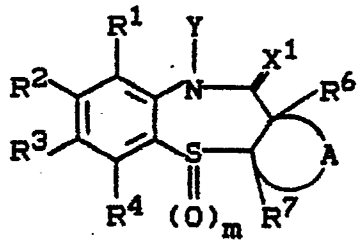

(VI)

oder

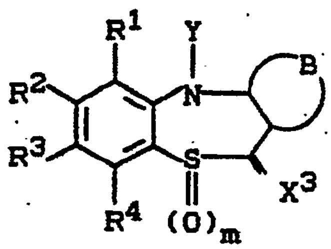

(VII)

oder

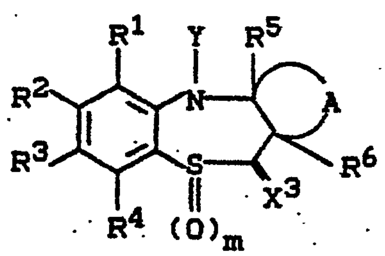

(VIII),

worin

$R^1$, $R^2$, $R^3$, $R^4$ gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxylamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen oder untereinander einen oder mehrere gesättigte oder ungesättigte, gegebenenfalls substituierte isocyclische oder heterocyclische Kohlenstoffringe mit bis zu 8 C-Atomen bilden,

Y Wasserstoff, gegebenenfalls substituiertes Alkyl, Aryl, Heteroaryl, Acyl, Thioacyl, Acyloxy, Arylamino oder Acylamino bedeutet,
$X^1$, $X^2$ oder $X^3$ folgende Gruppierungen bedeutet:

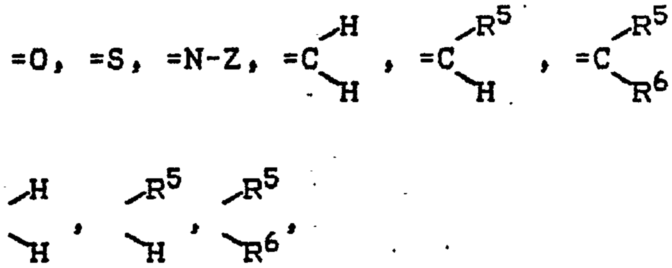

$R^5$, $R^6$ und $R^7$ gleich oder verschieden sind und die Bedeutung von $R^1$ bis $R^4$ besitzen, mit der Ausnahme, daß sie untereinander keinen cyclischen Ring bilden und
Z die Bedeutung von Y hat mit der Ausnahme, daß Z nicht gleich H ist,
A die Anellierung eines gegebenenfalls substituierten gesättigten isocyclischen oder heterocyclischen Rings mit bis zu 8 C-Atomen bedeutet,
B die Anellierung eines gegebenenfalls substituierten ungesättigten isocyclischen oder heterocyclischen Rings mit bis zu 8 C-Atomen bedeutet und
m 0 oder 1 bedeutet.

Bevorzugt werden eingesetzt Thiazepine der Formel:

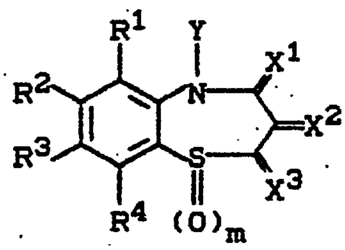

(II)

oder

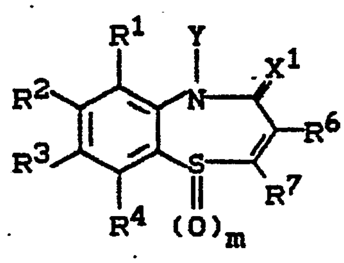

(III)

oder

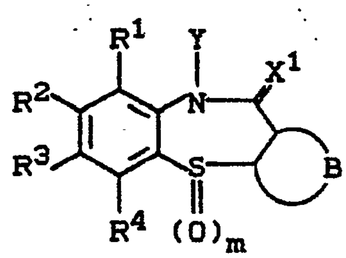

(V)

oder

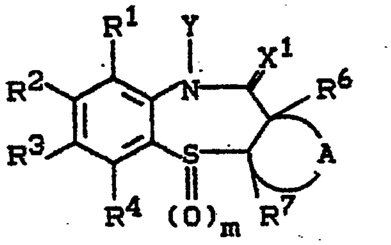

(VI)

Besonders bevorzugt sind Thiazepine der Formeln:

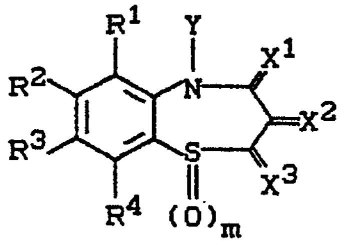

(II)

und

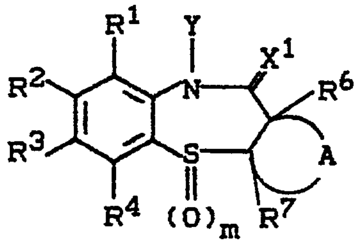

(VI).

Als Substituenten der zuvor genannten Reste seien genannt: Halogen, Nitro, Alkoxy, Alkyl, Aryl und Heteroaryl, bevorzugt Halogen und Alkyl.

Als Alkylreste seien solche mit 1 bis 16 C-Atomen, bevorzugt 1 bis 4 C-Atomen genannt, beispielsweise Methyl, Ethyl, n-Propyl, Isopropyl, n-Butyl, Isobutyl, t-Butyl, n-Pentyl-Cyclopentyl, n-Hexyl, Cyclohexyl, n-Decyl, n-Tridedecyl, n-Hexadecyl, Benzyl, Chlormethyl, Dichlormethyl, Trichlormethyl, Trifluormethyl, bevorzugt Methyl, n-Propyl, n-Tridecyl, Benzyl, Trifluormethyl;
als Arylreste seien solche mit 1 bis 10 C-Atomen, bevorzugt 1 bis 7 C-Atomen genannt, beispielsweise Phenyl, Naphthyl, Tolyl, Anisyl, Chlorphenyl, Nitrophenyl, bevorzugt Phenyl und Chlorphenyl;
als Heteroarylreste seien solche mit 1 bis 9 C-Atomen, bevorzugt 1 bis 5 C-Atomen, genannt, beispielsweise Pyridyl, Methylpyridyl, Furyl, Pyrrolyl, Imidazolyl, Thienyl, Indolyl, bevorzugt Furyl und Imidazolyl;
als Alkoxyreste seien solche mit 1 bis 6 C-Atomen, bevorzugt 1 bis 4 C-Atomen, genannt, beispielsweise Methoxy, Ethoxy, t-Butoxy, Cyclohexyloxy, Trifluormethoxy, bevorzugt Methoxy, Ethoxy;
als Aryloxyreste seien solche mit 1 bis 10 C-Atomen, bevorzugt 1 bis 7 C-Atomen, genannt, beispielsweise Phenoxy, Naphthoxy, Methylphenyloxy, Chlorphenyloxy, bevorzugt Phenoxy und Chlorphenyloxy;
als Heteroaryloxyreste seien solche mit 1 bis 9 C-Atomen, bevorzugt 1 bis 5 C-Atomen, genannt, beispielsweise Pyridyloxy, Furyloxy, Thienyloxy, bevorzugt Furyloxy;
als Acyloxyreste seien solche mit 1 bis 7 C-Atomen, bevorzugt 1 bis 4 C-Atomen, genannt, beispielsweise Acetyloxy, Formyloxy, Benzoyloxy, Trichloracetyloxy, Chlorphenylcarbonyloxy, Trifluoracetyloxy, bevorzugt Acetyloxy, Trichloracetyloxy;
als Alkylthioreste seien solche mit 1 bis 6 C-Atomen, bevorzugt 1 bis 4 C-Atomen, genannt, beispielsweise Methylthio, Ethylthio, Cyclohexylthio, Trifluormethylthio, Trichlormethylthio, bevorzugt Methylthio,
als Arylthioreste seien solche mit 1 bis 7 C-Atomen, bevorzugt 1 bis 6 C-Atomen, genannt, beispielsweise Phenylthio, Tolylthio, Anisylthio, Chlorphenylthio, bevorzugt Phenylthio und Chlorphenylthio;
als Heteroarylthioreste seien solche mit 1 bis 5 C-Atomen, bevorzugt 1 bis 4 C-Atomen, genannt, beispielsweise Pyridylthio, Furylthio, Imidazolylthio, bevorzugt Furylthio;
als Acylthioreste seien solche mit 1 bis 7 C-Atomen, bevorzugt 1 bis 4 C-Atomen, genannt, beispielsweise Benzoylthio, Acetylthio, Formylthio, Trichloracetylthio, Trifluoracetylthio, bevorzugt Acetylthio, Trichloracetylthio, Trifluoracetylthio;

als Acylreste seien solche mit 1 bis 7 C-Atomen, bevorzugt 1 bis 4 C-Atomen, genannt, beispielsweise Acetyl, Formyl, Propionyl, Benzoyl, Phenylacetyl, Chloracetyl, Dichloracetyl, Trichloracetyl, Chlorphenylcarbonyl, Trifluoracety, Chlorcarbonyl, bevorzugt Acetyl, Formyl, Chloracetyl, Dichloracetyl, Trichloracetyl, Trifluoracetyl, Chlorcarbonyl;
als Thioacylreste seien solche mit 1 bis 7 C-Atomen, bevorzugt 1 bis 4 C-Atomen genannt, beispielsweise Thioacetyl, Thiobenzoyl, Trichlorthioacetyl, bevorzugt Thioacetyl;
als Acylaminoreste seien solche mit 1 bis 8 C-Atomen, bevorzugt 1 bis 7 C-Atomen, genannt, beispielsweise Formylamino, Acetylamino, Trichloracetylamino, Trifluoracetylamino, Benzoylamino, Tolylcarbonylamino, Chlorphenylcarbonylamino, bevorzugt Acetylamino, Trifluoracetylamino, Bnzoylamino.

Als gesättigte oder ungesättigte, gegebenenfalls substituierte isocyclische oder heterocyclische Kohlenstoffringe (O, S und N-Heteroatome) mit bis zu 8 Kohlenstoffatomen seien genannt: Benzo, Naphthaleno, Thieno, Furano, Pyrrolo, Pyridino, Cyclohexano, Cyclopentano, Oxolano, Dioxolano, bevorzugt Benzo und Cyclohexano.

Als Halogene werden genannt Fluor, Chlor, Brom, Jod, bevorzugt Fluor, Chlor, Brom, besonders bevorzugt Chlor.

Besonders bevorzugte Reste $R^1$ bis $R^4$ sind Wasserstoff, Methyl, n-Propyl, n-Tridecyl, Benzyl, Trifluormethyl, Phenyl, Furyl, Methoxy, Phenoxy, Acetyloxy, Methylthio, Phenylthio, Acetyl, Formyl, Chlorcarbonyl, Nitro, Chlor sowie die Verbindungen der Reste untereinander in einem Benzolring.

Ganz besonders bevorzugt für die Reste $R^1$ - $R^4$ sind Wasserstoff, Methyl, Chlor und Methoxy.

Als bevorzugte Reste für Y seien genannt:
Wasserstoff, Methyl, Ethyl, n-Propyl, Isopropyl, n-Butyl, Isobutyl, tert.-Butyl, Benzyl, Phenyl, Formyl, Acetyl, Benzoyl, Trifluoracetyl und Trichloracetyl.

Ganz besonders bevorzugt für Y seien genannt: Wasserstoff, Methyl und Acetyl.

Als bevorzugt für die cyclischen Reste A seien genannt: Cyclohexano und Cyclopentano.

Als bevorzugt für die cyclischen Reste B seien genannt: Benzo und Naphthaleno.

Als Beispiele für die erfindungsgemäß einzusetzenden Thiazepine seien namentlich genannt:

2,3-Dihydro-5H-benzo[b]-1,4]thiazepin-4-on,
2-Methyl-2,3-dihydro-5H-benzo[b][1,4]thiazepin-4-on,
2-Ethyl-2,3-dihydro-5H-benzo[b][1,4]thiazepin-4-on,
2-Propyl-2,3-dihydro-5H-benzo[b][1,4]thiazepin-4-on,
3-Methyl-2,3-dihydro-5H-benzo[b][1,4]thiazepin-4-on,
2,3-Dimethyl-2,3-dihydro-5H-benzo[b][1,4]thiazepin-4-on,
2,2-Dimethyl-2,3-dihydro-5H-benzo[b][1,4]thiazepin-4-on,
2-Phenyl-2,3-dihydro-5H-benzo[b][1,4]thiazepin-4-on,
5-Methyl-2,3-dihydro-5H-benzo[b][1,4]thiazepin-4-on,
5-Benzyl-2,3-dihydro-5H-benzo[b][1,4]thiazepin-4-on,
5-Acetyl-2,3-dihydro-5H-benzo[b][1,4]thiazepin-4-on,
5-Chlorocarbonyl-2,3-dihydro-5H-benzo[b][1,4]thiazepin-4-on,
2-Methyl-5-acetyl-2,3-dihydro-5H-benzo[b][1,4]thiazepin-4-on,
2-Chlor-2,3-dihydro-5H-benzo[b][1,4]thiazepin-4-on,
2,3-Dichlor-2,3-dihydro-5H-benzo[b][1,4]thiazepin-4-on,
2,2,3-Trichlor-2,3-dihydro-5H-benzo[b][1,4]thiazepin-4-on,
2,2,3,3-Tetrachlor-2,3-dihydro-5H-benzo[b][1,4]thiazepin-4-on,
5-Methyl-2,3-dichlor-2,3-dihydro-5H-benzo[b][1,4]thiazepin-4-on,
3-Methoxy-2,3-dihydro-5H-benzo[b][1,4]thiazepin-4-on,
7-Chlor-2,3-dihydro-5H-benzo[b][1,4]thiazepin-4-on,
6,8-Dichlor-2,3-dihydro-5H-benzo[b][1,4]thiazepin-4-on,
6,8-Dichlor-2,3-dimethyl-2,3-dihydro-5H-benzo[b][1,4]thiazepin-4-on,
7-Chlor-5-acetyl-2,3-dihydro-5H-benzo[b][1,4]thiazepin-4-on,
7-Methyl-2,3-dihydro-5H-benzo[b][1,4]thiazepin-4-on,
7-Trifluormethyl-2,3-dihydro-5H-benzo[b][1,4]thiazepin-4-on,
6,8-Dimethyl-2,3-dihydro-5H-benzo[b][1,4]thiazepin-4-on,
2,3-Dihydro-5H-[2,3]napthaleno[b][1,4]tiazepin-4-on,
5H-Dibenzo[b,f][1,4]thiazepin-4-on,
kernchloriertes 5H-Dibenzo[b,f][1,4]thiazepin-4-on,
2,3-Tetramethylen-2,3-dihydro-5H-benzo[b][1,4]thiazepin-4-on,
6,8-Dimethyl-2,3-tetramethylen-2,3-dihydro-5H-benzo[b] [1,4]thiazepin-4-on,
3H-5H-Benzo[b][1,4]thiazepin-2,4-dion,
3H-5H-Benzo[b][1,4]thiazepin-2-on-4-thion,
3H-5H-Benzo[b][1,4]thiazepin-2,4-dithion,
2,3-Dihydro-5H-benzo[b][1,4]thiazepin-4-thion,
2-Methyl-2,3-dihydro-5H-benzo[b][1,4]thiazepin-4-thion,
6,8-Dichlor-2,3-dihydro-5H-benzo[b][1,4]thiazepin-4-thion,
5-Acetyl-2,3-dihydro-5H-benzo[b]thiazepin-4-thion,

8-Acetyl-2,3-dihydro-5H-benzo[b][1,4]thiazepin-4-on,
1-Oxo-2,3-dihydro-5H-1λ$^4$-benzo[b][1,4]thiazepin-4-on,
1-Oxo-2-methyl-2,3-dihydro-5H-1λ$^4$ benzo[b][1,4]thiazepin-4-on,
1-Oxo-7-chlor-5-methyl-2,3-dihydro-5H-1λ$^4$-benzo[b][1,4]thiazepin-4-thion,
1-Oxo-2,3-dimethyl-2,3-dihydro-5H-1λ$^4$-benzo[b][1,4]thiazepin-4-on,
5,8-Diacetyl-2-methyl-2,3-dihydro-5H-benzo[b][1,4]thiazepin-4-on,
5H-Benzo[b][1,4]thiazepin-4-on,
2-Chlor-5H-benzo[b][1,4]thiazepin-4-on,
2,3-Dichlor-5H-benzo[b][1,4]thiazepin-4-on,
2,3,7-Trichlor-5H-benzo[b][1,4]thiazepin-4-on,
2,3,8-Trichlor-5H-benzo[b][1,4]thiazepin-4-on,
5-Acetyl-5H-benzo[b][1,4]thiazepin-4-on,
2-Phenyl-5H-benzo[b][1,4]thiazepin-4-on,
2-Methyl-5H-benzo[b][1,4]thiazepin-4-on,
1-Oxo-2,3-dihydro-5H-1λ$^4$-[2,3]naphthaleno[b][1,4]thiazepin-4-on,
5-Acetyl-4,5-dihydro-3H-benzo[b][1,4]thiazepin-2-on,
7-Chlor-5-acetyl-4,5-dihydro-3H-benzo[b][1,4]thiazepin-2-on,
3-Acetoxy-2,3-dihydro-5H-benzo[b][1,4]thiazepin-4-on,
3-Acetoxy-2-phenyl-2,3-dihydro-5H-benzo[b][1,4]thiazepin-4-on,
3-Acetoxy-2-(4-methoxyphenyl)-2,3-dihydro-5H-benzo[b] [1,4]thiazepin-4-on,
3-Acetamino-2,3-dihydro-5H-benzo[b][1,4]thiazepin-4-on,
3-Acetamino-2-phenyl-2,3-dihydro-5H-benzo[b][1,4]thiazepin-4-on,
8-Chlor-3-acetoxy-2,3-dihydro-5H-benzo[b][1,4]thiazepin-4-on,
8-Chlor-3-acetoxy-2-(4-methoxyphenyl)-5H-benzo[b][1,4]thiazepin-4-on,
5[2-(Dimethylamino)ethyl]-3-acetoxy-2-(4-methoxyphenyl)-5H-benzo[b][1,4]thiazepin-4-on,
2,3,4,5-Tetrahydro-benzo[b][1,4]thiazepin,
5-Acetyl-2,3,4,5-tetrahydro-benzo[b][1,4]thiazepin
2,3-Dimethyl-2,3,4,5-tetrahydro-benzo[b][1,4]thiazepin
5-Ethyl-2,3,4,5-tetrahydro-benzo[b][1,4]thiazepin,
2-(2-Aminophenylthio)-2,3-dihydro-5H-benzo[b][1,4] thiazepin-4-on,
2-n-Tridecyl-2,3-dihydro-5H-benzo[b][1,4]thiazepin-4-on,
5-n-Pentyl-2,3-dihydro-5H-benzo[b][1,4]thiazepin-4-on,
2-(2-Furyl)-2,3-dihydro-5H-benzo[b][1,4]thiazepin-4-on,
2-[3(4)-Imidazolyl]-2,3-dihydro-5H-benzo[b][1,4]thiazepin-4-on,
5-Phenyl-2,3-dihydro-5H-benzo[b][1,4]thiazepin-4-on,
8-Methoxy-2,3-dihydro-5H-benzo[b][1,4]thiazepin-4-on,
8-Ethoxy-2,3-dihydro-5H-benzo[b][1,4]thiazepin-4-on,
8-Nitro-2,3-dihydro-5H-benzo[b][1,4]thiazepin-4-on,
7,8,9-Trichlor-2,3dihydro-5H-benzo[b][1,4]thiazepin-4-on,
7-Sulfoxyl-2,3-dihydro-5H-benzo[b][1,4]thiazepin-4-on,
8-Acetamino-2,3-dihydro-5H-benzo[b][1,4]thiazepin-4-on,
8-Methyl-2,3-dihydro-5H-benzo[b][1,4]thiazepin-4-on,
8-Chlor-6-methyl-2,3-dihydro-5H-benzo[b][1,4]thiazepin-4-on,
6,7,8,9-Tetrachlor-2,3-dihydro-5H-benzo[b][1,4]thiazepin-4-on,
4,5-Dihydro-2H-benzo[b][1,4]thiazepin-3-on,
7,9-Dimethoxy-2,3-dihydro-5H-benzo[b][1,4]thiazepin-4-on,
7,9-Dimethyl-2,3-dihydro-5H-benzo[b][1,4]thiazepin-4-on,
7,8-Dimethyl-2,3-dihydro-5H-benzo[b][1,4]thiazepin-4-on,
7-Methoxy-2,3-dihydro-5H-benzo[b][1,4]thiazepin-4-on,
7,9-Dimethyl-2,3-tetramethylen-2,3-dihydro-5H-benzo[b][1,4]thiazepin-4-on,
5-Trifluoracetyl-2,3,4,5-tetrahydro-benzo[b][1,4]thiazepin,
5-Trifluoracetyl-5H-dibenzo[b,f][1,4]thiazepin,
2,3-Dihydro-5H-[1,2]naphthaleno[b][1,4]thiazepin-4-on,
2,3,7,9-Tetramethyl-2,3-dihydro-5H-benzo[b][1,4]thiazepin-4-on,
bevorzugt:
2,3-Dihydro-5H-benzo[b][1,4]thiazepin-4-on,
2-Methyl-2,3-dihydro-5H-benzo[b][1,4]thiazepin-4-on,
2-Ethyl-2,3-dihydro-5H-benzo[b][1,4]thiazepin-4-on,
2-Propyl-2,3-dihydro-5H-benzo[b][1,4]thiazepin-4-on,
3-Methyl-2,3-dihydro-5H-benzo[b][1,4]thiazepin-4-on,
2,3-Dimethyl-2,3-dihydro-5H-benzo[b][1,4]thiazepin-4-on,
5-Methyl-2,3-dihydro-5H-benzo[b][1,4]thiazepin-4-on,
5-Benzyl-2,3-dihydro-5H-benzo[b][1,4]thiazepin-4-on,

5-Acetyl-2,3-dihydro-5H-benzo[b][1,4]thiazepin-4-on,

5-Chlorocarbonyl-2,3-dihydro-5H-benzo[b][1,4]thiazepin-4-on,
2-Methyl-5-acetyl-2,3-dihydro-5H-benzo[b][1,4]thiazepin-4-on,
7-Chlor-2,3-dihydro-5H-benzo[b][1,4]thiazepin-4-on,
6,8-Dichlor-2,3-dihydro-5H-benzo[b][1,4]thiazepin-4-on,
6,8-Dichlor-2,3-dimethyl-2,3-dihydro-5H-benzo[b][1,4]thiazepin-4-on,
7-Methyl-2,3-dihydro-5H-benzo[b][1,4]thiazepin-4-on,
7-Trifluormethyl-2,3-dihydro-5H-benzo[b][1,4]thiazepin-4-on,
6,8-Dimethyl-2,3-dihydro-5H-benzo[b][1,4]thiazepin-4-on,
2,3-Dihydro-5H-[2,3]naphthaleno[b][1,4]thiazepin-4-on,
5H-Dibenzo[b,f][1,4]thiazepin-4-on,
kernchloriertes 5H-Dibenzo[b,f][1,4]thiazepin-4-on,
2,3-Tetramethylen-2,3-dihydro-5H-benzo[b][1,4]thiazepin-4-on,
6,8-Dimethyl-2,3-tetramethylen-2,3-dihydro-5H-benzo[b][1,4]thiazepin-4-on,
3H-5H-Benzo[b][1,4]thiazepin-2,4-dion,
3H-5H-Benzo[b][1,4]thiazepin-2-on-4-thion,
3H-5H-Benzo[b][1,4]thiazepin-2,4-dithion,
2,3-Dihydro-5H-benzo[b][1,4]thiazepin-4-thion,
2-Methyl-2,3-dihydro-5H-benzo[b][1,4]thiazepin-4-thion,
5-Acetyl-2,3-dihydro-5H-benzo[b][1,4]thiazepin-4-thion,
1-Oxo-2,3-dihydro-5H-1$\lambda^4$-benzo[b][1,4]thiazepin-4-on,
1-Oxo-7-chlor-5-methyl-2,3-dihydro-5H$\lambda^4$-benzo[b][1,4]thiazepin-4-thion,
1-Oxo-2,3-dimethyl-2,3-dihydro-5H-1$\lambda^4$-benzo[b][1,4]thiazepin-4-on,
3-Acetoxy-2,3-dihydro-5H-benzo[b][1,4]thiazepin-4-on,
3-Acetamino-2,5-dihydro-5H-benzo[b][1,4]thiazepin-4-on,
2,3,4,5-Tetrahydro-benzo[b][1,4]thiazepin,
2,3-Dimethyl-2,3,4,5-tetrahydro-benzo[b][1,4]thiazepin,
5-Ethyl-2,3,4,5-tetrahydro-benzo[b][1,4]thiazepin,
2-n-Tridecyl-2,3-dihydro-5H-benzo[b][1,4]thiazepin-4-on,
5-n-Pentyl-2,3-dihydro-5H-benzo[b][1,4]thiazepin-4-on,
8-Methoxy-2,3-dihydro-5H-benzo[b][1,4]thiazepin-4-on,
8-Ethoxy-2,3-dihydro-5H-benzo[b][1,4]thiazepin-4-on,
8-Methyl-2,3-dihydro-5H-benzo[b][1,4]thiazepin-4-on,
8-Chlor-6-methyl-2,3-dihydro-5H-benzo[b][1,4]thiazepin-4-on,
7,9-Dimethoxy-2,3-dihydro-5H-benzo[b][1,4]thiazepin-4-on,
7,9-Dimethyl-2,3-dihydro-5H-benzo[b][1,4]thiazepin-4-on,
7,8-Dimethyl-2,3-dihydro-5H-benzo[b][1,4]thiazepin-4-on,
7-Methoxy-2,3-dihydro-5H-benzo[b][1,4]thiazepin-4-on,
7,9-Dimethyl-2,3-tetramethylen-2,3-dihydro-5H-benzo[b][1,4]thiazepin-4-on,
2,3-Dihydro-5H-[1,2]naphthaleno[b][1,4]thiazepin-4-on,
2,3,7,9-Tetramethyl-2,3-dihydro-5H-benzo[b][1,4]thiazepin-4-on.

Die Herstellung der erfindungsgemäßen Co-Katalysatoren ist aus der Literatur allgemein bekannt. Man kann sie auf einfache Weise z.B. in einem Schritt aus gegebenenfalls substituiertem o-Aminothiophenol durch Erhitzen mit gegebenenfalls substituierter Acrylsäure (J. Chem. Soc. 1927, 2738) oder $\alpha,\beta$-ungesättigten Ketonen (Chem. Ber. 90, 2683 (1957)) herstellen. Weiterhin sind sie durch Cyclisierung von gegebenenfalls substituierten 2-(o-Aminophenylmercapto)-propionsäuren leicht erhältlich (Ber. 56, 1415 (1923)).

Als aromatische Kohlenwasserstoffe die erfindungsgemäß eingesetzt werden können, werden bevorzugt genannt: Toluol, Ethylbenzol, Propylbenzol, Cumol, tert.-Butyl-benzol und Phenylcyclohexan. Bevorzugt ist Toluol.

Das erfindungsgemäße Verfahren wird in flüssiger Phase durchgeführt, wobei der aromatische Kohlenwasserstoff in flüssiger Form oder gegebenenfalls in Verdünnung mit einem inerten Lösungsmittel eingesetzt werden kann. Bevorzugt ist die flüssige unverdünnte Form.

Nach dem erfindungegemäßen Verfahren wird als Chlorierungsmittel vorzugsweise Chlor verwendet. Das Chlor wird flüssig oder gasförmig in das Reaktionsgemisch geleitet.

Bevorzugt ist gasförmiges Chlor. Es können aber auch andere Chlorierungsmittel als Chlor verwendet werden. Dies sind chlorhaltige Verbindungen, die unter den Reaktionsbedingungen Chlor abgeben. Als Beispiel sei Sulfurylchlorid genannt.

Als Reaktionsdruck ist Unter-, Über- oder Normaldruck möglich, bevorzugt ist Normaldruck.

Der Chlorierungsgrad liegt bevorzugt nicht höher als 1. Höhere Chlorierungsgrade sind möglich, aber gewöhnlich nicht vorteilhaft, da sie zur Bildung unerwünschter mehrfach chlorierter Produkte führen.

Die Reaktion kann bei Temperaturen vom Erstarrungspunkt bis zum Siedepunkt des Gemisches durchgeführt werden. Die Reaktionstemperatur liegt gewöhnlich bei 0 bis 100°C, bevorzugt sind 20-80°C, besonders bevorzugt 40-60°C.

Erfindungsgemäß als Friedel-Crafts-Katalysatoren einsetzbar sind alle bekannten Friedel-Crafts-Katalysatoren. Beispielsweise seien folgende Friedel-Crafts-Katalysatoren genannt: Antimonchloride, Antimonoxychloride, Aluminiumchlorid, Eisen(II)-chlorid, Eisen(III)-chlorid, Tellurchloride, Molybdänchloride, Wolframchloride, Titanchloride, Zinkchloride, Zinnchloride, Borchloride und/oder Bortrifluorid.

Weiterhin umfassen die erfindungsgemäß einsetzbaren Friedel-Crafts-Katalysatoren auch Elements und Elementverbindungen, die während der Chlorierung eine Lewis- Säure bilden oder die Funktion einer Lewis-Säure ausüben, z.B. Antimon, Eisen, Blei, Zinn, Zink, Molybdän, Tellur und Aluminium oder Oxide, Sulfide oder Carbonyle derselben sowie borhaltige Verbindungen, wie Antimonoxide, Eisenoxide, Eisensulfide, Bleisulfide, Zinnsulfide, Zinksulfide, Eisencarbonyle, Molybdäncarbonyle und/oder Borphosphat. Erfindungsgemäß können anstelle der erwähnten Chloride auch die Bromise und gegebenenfalls auch die Fluorise oder Jodide der genannten Elemente eingesetzt werden. Bevorzugt sind Antimonchloride, Aluminiumchlorid, Eisen, Eisenoxide, Eisensulfide, Eisencarbonyle und/oder Eisen(III)-chlorid. Besonders bevorzugt ist Eisen(III)-chlorid.

Die Mengen, in denen die erfindungsgemäß zu verwendenden Friedel-Crafts-Katalysatoren eingesetzt werden, können in weiten Grenzen variiert werden. Bereits bei Zusätzen von 0,0005 Gew.-% macht sich die Katalysatorwirkung bemerkbar. Es können auch 5 Gew.-% oder mehr Friedel-Crafts-Katalysator zugesetzt werden, jedoch bieten diese hohen Mengen im allgemeinen keinen Vorteil.

Üblicherweise werden die Friedel-Crafts-Katalysatoren in einer Menge von etwa 0,001 bis 0,5 Gew.-%, bevorzugt von 0,01 bis 0,10 Gew.-%, bezogen auf das eingesetzte Toluol, zugegeben.

Zu den erfindungsgemäß einsetzbaren Co-Katalysatoren gehören weiterhin alle Substanzen, die unter den Reaktionsbedingungen eine Verbindung oder Gemische von Verbindungen bilden können, die durch die genannten Formeln der erfindungsgemäßen Co-Katalysatoren beschrieben wer den. Das sind insbesondere solche Verbindungen, die in dem Siebenring mehrfach ungesättigt sind. Weiterhin sind es offenkettige Vorstufen, die durch Ringschluß in die erfindungsgemäßen Co-Katalysatoren übergehen können.

Zu den erfindungsgemäß einsetzbaren Co-Katalysatoren gehören weiterhin alle Substanzen, die durch Reaktion der zuvor genannten erfindungsgemäßen Co-Katalysatoren mit Chlor oder Chlorwasserstoff unter den Reaktionsbedingungen der Chlorierung der aromatischen Kohlenwasserstoffe gebildet werden können. Insbesondere gilt dies für die Hydrochloride der zuvor genannten erfindungsgemäßen Co-Katalysatoren.

Es ist auch möglich, die Co-Katalysatoren in Kombination mit anderen nicht als Co-Katalysator beanspruchten Elementen oder Verbindungen in das erfindungsgemäße Verfahren einzusetzen. Die Co-Katalysatoren können sowohl einzeln als auch im Gemisch untereinander eingesetzt werden.

Die Mengen, in denen die erfindungsgemäßen Co-Katalysatoren eingesetzt werden, können in weiten Grenzen variiert werden. Mengen unter 0,0001 Gew.-% sind jedoch im allgemeinen nicht vorteilhaft, da dann die co-katalytische Wirkung nicht mehr bemerkbar ist. Es können auch 5 Gew.-% oder mehr Co-Katalysator zugesetzt werden, jedoch bieten diese hohen Mengen im allgemeinen keinen Vorteil. Üblicherweise werden die Co-Katalysatoren in einer Menge von etwa 0,0001 bis 0,5 Gew.-%, bevorzugt in einer Menge von 0,0005 bis 0,1 Gew.-%, ganz besonders bevorzugt in einer Menge von 0,001 bis 0,01 Gew.-%, bezogen auf den eingesetzten aromatischen Kohlenwasserstoff, eingesetzt.

Das Molverhältnis des Gemisches von mindestens einem Friedel-Crafts-Katalysator und mindestens einem Co-Katalysator kann gemäß dem erfindungsgemäßen Verfahren in weiten Grenzen variiert werden. Im allgemeinen ist es nicht vorteilhaft, den Co-Katalysator in großem Überschuß gegenüber dem Friedel-Crafts-Katalysator einzusetzen, da sonst die Chlorierungsreaktion gehemmt wird. Ebenso ist es im allgemeinen nicht vorteilhaft, den Überschuß an Friedel-Crafts-Katalysator zu groß zu wählen, da sonst die selektivitätssteuernde Wirkung der Co-Katalysatoren nicht in Erscheinung tritt. Üblicherweise arbeitet man bei der Ausführung des erfindungsgemäßen Verfahrens bei einem molaren Verhältnis von Friedel-Crafts-Katalysator zu Co-Katalysator von etwa 100:1 bis 1:10, bevorzugt ist das molare Verhältnis von Friedel-Crafts-Katalysator zu Co-Katalysator von 50:1 bis 1:4, besonders bevorzugt ist das molaire Verhältnis Friedel-Crafts-Katalysator zu Co-Katalysator von 20:1 bis 1:2.

Bei der praktischen Ausführung des erfindungsgemäßen Verfahrens ist die Reihenfolge der Zugabe der einzelnen Komponenten des katalytischen Systems aus Friedel-Crafts-Katalysatoren und Co-Katalysatoren beliebig. Dabei ist das Verfahren sowohl kontinuierlich als auch diskontinuierlich durchführbar. Das erfindungsgemäße Verfahren wird z.B. wie folgt durchgeführt:

Der gewünschte aromatische Kohlenwasserstoff, z.B. Toluol, wird vorgelegt und auf die gewünschte Temperatur, z.B. 50°C, gebracht. Dann gibt man in beliebiger Reihenfolge die gewünschten Mengen an Friedel-Crafts-Katalysatoren und Co-Katalysatoren zu und leitet unter weitgehender Konstanthaltung der Temperatur Chlor gasförmig bis zum gewünschten Chlorierungsgrad ein. Anschließend wird das Gemisch wie üblich durch Destillation aufgearbeitet. Ebenfalls erfindungsgemäß ist folgende Verfahrensweise: Man stellt eine Mischung aus Alkylbenzol mit den gewünschten Anteilen an Katalysator und Co-Katalysator her und bringt diese auf die gewünschte Reaktionstemperatur. Dann leitet man ein Chlorierungsmittel bis zum gewünschten Chlorierungsgrad ein. Die Aufarbeitung erfolgt wie üblich durch Destillation. Ebenfalls erfindungsgemäß ist auch folgende Verfahrensweise: Man stellt eine Lösung der gewünschten Friedel-Crafts-Katalysatoren mit den Co-Katalysatoren in dem Alkylbenzol her und führt diese einer kontinuierlich arbeitenden Chlorierapparatus zu. Man leitet ein Chlorierungsmittel so schnell ein, daß der gewünschte Chlorierungsgrad erreicht ist. Die kontinuierlich anfallende Reaktionsmischung wird wie üblich durch Destillation aufgearbeitet.

Bei dem erfindungsgemäßen Verfahren ist überraschend, daß die erfindungsgemäßen Co-Katalysatoren, d.h. die Verbindungen der angegebenen allgemeinen Formeln eine so ausgeprägte co-katalytische Wirkung aufweisen, d.h. daß sie in Kombination mit den Friedel-Crafts-Katalysatoren in der Lage sind, das o/p-Verhältnis der Chlorierung von aromatischen Kohlenwasserstoffen derart zu beeinflussen, daß überwiegend die p-Verbindung gebildet wird. Bisher bekannte Heterocyclen zur Steuerung der o/p-Selektivität hatten immer die Form von drei linear annellierten Sechsringen, d.h. sie hatten also eine völlig andere Struktur. Weiterhin ist ausgesprochen überaschend, daß die erfindungsgemäßen Co-Katalysatoren gerade mit dem technisch außerodentlich günstigen Friedel-Crafts-Katalysator Eisen(III)-chlorid so gute Ergebnisse liefern.

Ebenso überraschend ist, daß diese guten Ergebnisse bei technischen sehr vorteilhaften Temperaturen z.B. 40 bis 60°C erreicht werden. Ferner ist ausgesprochen überraschend, daß die erfindungsgemäßen Co-Katalysatoren ihre p-selektive Wirkung bereits bei äußerst geringen Konzentrationen zeigen, so daß die notwendigen Einsatzmengen an Co-Katalysatoren besonders gering sind. Sie liegen im ganz besonders bevorzugten Bereich von 0,001 bis 0,01 Gew.-% um Zehnerpotenzen niedriger als bei bisher bekannten Co-Katalysatoren. Diese Tatsache ist technisch wie ökonomisch und ökologisch außerodentlich vorteilhaft.

Das nach dem erfindungsgemäßen Verfahren für Toluol erreichbare o/p-Verhältnis von 0,64 ist das niedrigste o/p-Verhältnis, das bisher bei einer Eisen(III)-chlorid-Katalyse und bei mittleren Reaktionstemperaturen von 40 bis 60°C erreicht wurde.

Weiterhin ist technisch außerordentlich vorteilhaft, daß die erfindungsgemäßen Co-Katalysatoren auf einfach Art durch einen einzigen Reaktionsschritt aus technisch verfügbaren Ausgangsmaterialien herstellbar sind.

Die folgenden Beispiele sollen das erfindungsgemäße Verfahren verdeutlichen, ohne es jedoch auf diese Beispiele einzuschränken.

Beispiel 1

Man legte 100 Gew.-Teile Toluol in einem Reaktor vor und gab unter Rühren 0,0175 Gew.-Teile $FeCl_3$ und 0,004 Gew.-Teile des Co-Katalysators der Formel

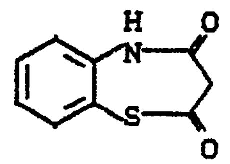

(3H-5H-Benzo[b][1,4] thiazepin-2,4-dion)

zu und erhitzte auf 55°C. Unter weitgehender Konstanthaltung der Temperatur leitete man ca. 94 mol-% Chlor gasförmig im Verlaufe von 5h gleichmäßig ein. Der Restgehalt an Toluol betrug im Reaktionsgemisch 3,3 Gew.-%, das Verhältnis von ortho-Chlortoluol zu para-Chlortoluol (o/p) betrug 0,75.

Beispiel 2

Das Verfahren von Beispiel 1 wurde wiederholt. Man gab jedoch 0,0045 Gew.-Teile des Co-Katalysators der Formel

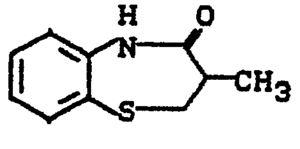

(3-Methyl-2,3-dihydro-5H-benzo[b][1,4]thiazepin-4-on)

zu. Der Toluolgehalt betrug 3,6 Gew.-%, das o/p-Verhältnis 0,75.

Beispiel 3

Das Verfahren von Beispiel 1 wurde wiederholt. Man gab jedoch 0,005 Gew.-Teile des Co-Katalysators der Formel

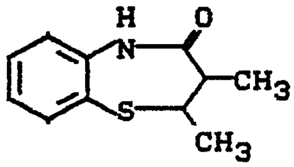

(2,3-Dimethyl-2,3-dihydro-5H-benzo-[b][1,4]thiazepin-4-on)

zu. Der Toluolgehalt betrug 3,0 Gew.-%, das o/p-Verhältnis 0,71.

Beispiel 4

Das Verfahren von Beispiel 1 wurde wiederholt. Man gab jedoch 0,009 Gew.-Teile des Co-Katalysators der Formel

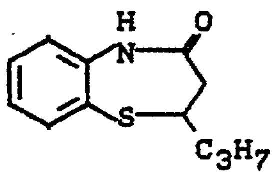

(2-Propyl-2,3-dihydro-5H-benzo[b][1,4]thiazepin-4-on)

zu. Der Toluolgehalt betrug 4,0 Gew.-%, das o/p-Verhältnis 0,74.

Beispiel 5

Das Verfahren von Beispiel 1 wurde wiederholt. Man gab jedoch 0,0025 Gew.-Teile des Co-Katalysators der Formel

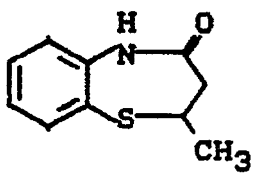

(2-Methyl-2,3-dihydro-5H-benzo[b][1,4]thiazepin-4-on)

zu und erhitzte auf 50°C. Der Toluolgehalt betrug 4,1 Gew.-% und das o/p-Verhältnis 0,74.

Beispiel 6

Das Verfahren von Beispiel 1 wurde wiederholt. Man gab jedoch 0,004 Gew.-Teile des Co-Katalysators der Formel

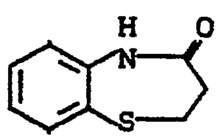

(2,3-Dihydro-5H-benzo[b][1,4]thiazepin-4-on

zu und erhitzte auf 40°C. Der Toluolgehalt betrug 3,4 Gew.-%, das o/p Verhältnis 0,74.

Beispiel 7

Das Verfahren von Beispiel 1 wurde wiederholt. Man gab jedoch 0,005 Gew.-Teile des Co-Katalysators der Formel

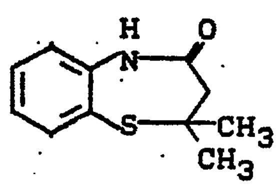

(2,2-Dimethyl-2,3-Dihydro-5H-benzo[b][1,4]thiazepin-4-on)

zu. Der Toluolgehalt betrug 6,9 Gew.-%, das o/p-Verhältnis 1,14.

Beispiel 8

Das Verfahren von Beispiel 1 wurde wiederholt. Man setzte jedoch 0,005 Gew.-Teile des Co-Katalysators der Formel

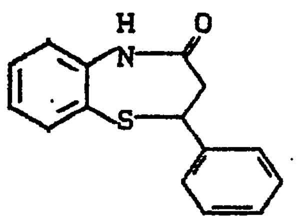

(2-Phenyl-2,3-dihydro-5H-benzo[b][1,4]thiazepin-4-on)

zu. Der Toluolgehalt betrug 7,2 % das o/p-Verhältnis 1,19.

Beispiel 9

Das Verfahren von Beispiel 1 wurde wiederholt. Man setzte jedoch 0,005 Gew.-Teile des Co-Katalysators der Formel

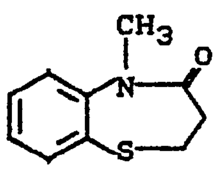

(5-Methyl-2,3-dihydro-5H-benzo[b][1,4]thiazepin-4-on)

zu und erhitzte auf 40°C. Der Toluolgehalt betrug 3,4 Gew.-%, das o/p-Verhältnis 0,84.

Beispiel 10

Das Verfahren von Beispiel 1 wurde wiederholt. Man setzte jedoch 0,005 Gew.-Teile des Co-Katalysators der Formel

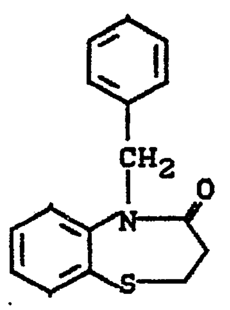

(5-Benzyl-2,3-dihydro-5H-benzo[b][1,4]thiazepin-4-on)

zu und erhitzte auf 50°C. Der Toluolgehalt betrug 5,5 Gew.-%, das o/p-Verhältnis 0,92.

Beispiel 11

Das Verfahren von Beispiel 1 wurde wiederholt. Man setzte jedoch 0,006 Gew.-Teile des Co-Katalysators der Formel

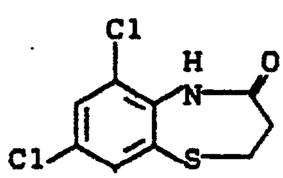

(6,8-Dichlor-2,3-dihydro-5H-benzo[b][1,4]thiazepin-4-on)

zu und erhitzte auf 50°C. Der Toluolgehalt betrug 4,4 Gew.-%, das o/p-Verhältnis 0,90.

Beispiel 12

Das Verfahren von Beispiel 1 wurde wiederholt. Man setzte jedoch 0,005 Gew.-Teile des Co-Katalysators der Formel

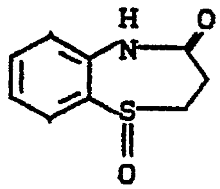

(1-Oxo-2,3-dihydro-5H-1λ4-benzo[b][1,4]thiazepin-4-on)

zu und erhitzte auf 50°C. Der Toluol betrug 4,3 Gew.-%, das o/p-Verhältnis 0,76.

Beispiel 13

Das Verfahren von Beispiel 1 wurde wiederholt. Man setzte jedoch 0,035 Gew.-Teile $FeCl_2$ und 0,06 Gew.-Teile des Co-Katalysators der Formel

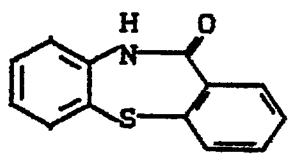

(5H-Dibenzo[b,f][1,4]thiazepin-4-on)

zu und erhitzte auf 50°C. Der Toluolgehalt betrug 5,0 Gew.-% und das o/p-Verhältnis 0,94.

Beispiel 14

Das Verfahren von Beispiel 1 wurde wiederholt, man setzte jedoch 0,035 Gew.-Teile $FeCl_3$ und 0,093 Gew.-Teile des Co-Katalysators der Formel

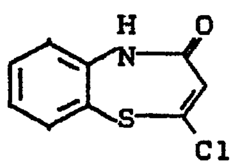

(2-Chlor-5H-benzo[b][1,4]thiazepin-4-on)

zu. Der Toluolgehalt betrug 8,1 Gew.-% und das o/p-Verhältnis 1,06.

Beispiel 15

Das Verfahren von Beispiel 1 wurde wiederholt. Man setzte jedoch 0,0046 Gew.-Teile des Co-Katalysators der Formel

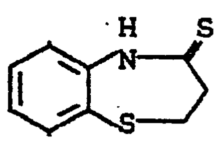

(2,3-Dihydro-5H-benzo[b][1,4]thiazepin-4-thion)

zu und erhitzte auf 50°C. Der Tolulgehalt betrug 3,4 Gew.-%, das o/p-Verhältnis 0,76.

Beispiel 16

Das Verfahren von Beispiel 1 wurde wiederholt, man legte jedoch 100 Gew.-Teile Cumol vor und setzte 0,005 Gew.-Teile des Co-Katalysators der Formel

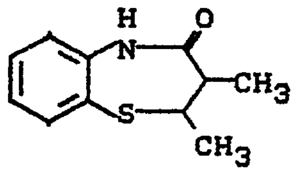

(2,3-Dimethyl-2,3-dihydro-5H-benzo[b][1,4]thiazepin-4-on)

zu und erhitzte auf 50°C. Der Gehalt an Cumol war 3,4 Gew.-%, das Verhältnis von ortho-Chlorisopropylbenzol zu para-Chlorisopropylbenzol 0,29.

Beispiel 17

Das Verfahren von Beispiel 1 wurde wiederholt. Man legte jedoch 100 Gew.-Teile Ethylbenzol vor und setzte 0,005 Gew.-Teile des Co-Katalysators der Formel

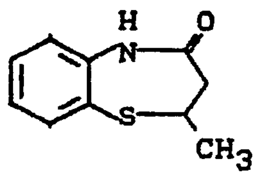

(2-Methyl-2,3-dihydro-5H-benzo[b][1,4]thiazepin-4-on)

zu und erhitzte auf 50°C. Der Gehalt an Ethylbenzol war 4,2 Gew.-%, das Verhältnis von ortho-Chlorethylbenzol zu para-Chlorethylbenzol 0,54.

Beispiel 18

Das Verfahren des Beispiels 1 wurde wiederholt. Man legte jedoch 100 Gew.-Teile t-Butylbenzol vor und setzte 0,0045 Gew.-Teile des Co-Katalysators der Formel

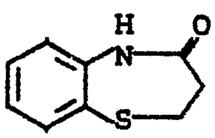

(2,3-Dihydro-5H-benzo[b][1,4]thiazepin-4-on)

zu und erhitzte auf 50°C. Der Gehalt an t-Butylbenzol betrug 8,9 Gew.-%, das Verhältnis von o-Chlor-t-butylbenzol zu para-Chlor-t-butylbenzol 0,17.

Beispiel 19

Das Verfahren des Beispiels 1 wurde wiederholt. Man legte jedoch 100 Gew.-Teile Cyclohexylbenzol vor, erhitzte auf 50°C und setzte 0,0045 Gew.-Teile des Co-Katalysators der Formel

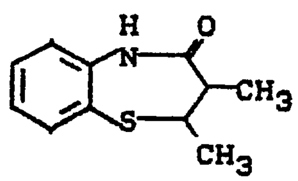

(2,3-Dimethyl-2,3-dihydro-5H-benzo[b][1,4]thiazepin-4-on)

zu. Der Gehalt an Cyclohexylbenzol betrug 2,6 Gew.-%, das Verhältnis ortho-Chlor-cyclohexylbenzol zu para-Chlor-cyclohexylbenzol 0,25.

Beispiel 20

Es wurde eine Lösung aus 100 Gew.-Teilen Toluol, 0,0175 Gew.-Teilen $FeCl_3$ und 0,004 Gew.-Teilen des Co-Katalysators der Formel

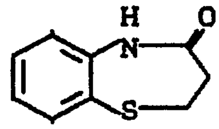

(2,3-Dihydro-5H-benzo[b][1,4] thiazepin-4-on)

bei Raumtemperatur hergestellt.

Diese Lösung führte man einem kontiniuerlich arbeitenden Chlorierreaktor bei 40-43°C zu, wobei gleichzeitig die äquivalente Menge an Chlorierungsprodukt entnommen wurde. Als Chlorierungsmittel wurde gasförmiges Chlor so schnell zugeführt, daß der Umsatz weitgehend konstant bei 90 mol-% lag. Das abgeführte Reaktionsgemisch enthielt 7,1 Gew.-% Toluol, das Verhältnis von ortho-Chlortoluol zu para-Chlortoluol war 0,70.

Beispiel 21

Das Verfahren von Beispiel 20 wurde wiederholt, man gab jedoch 0,0045 Gew.-Teile des Co-Katalysators der Formel

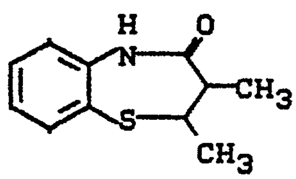

(2,3-Dimethyl-2,3-dihydro-5H-benzo[b][1,4]thiazepin-4-on)

zu. Das abgeführte Reaktionsgemisch enthielt 7,0 Gew.-% Toluol, das o/p-Verhältnis war 0,67.

Beispiel 22

Das Verfahren des Beispiels 20 wurde wiederhalt, man gab jedoch 0,005 Gew.-Teile des Co-Katalysators der Formel

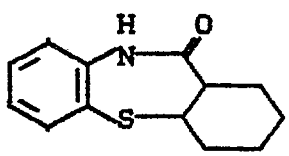

(2,3-Tetramethylen-2,3-dihydro-5H-benzo[b][1,4] thiazepin-4-on)

zu. Das abgeführte Reaktionsgemisch enthielt 7,0 Gew.-% Toluol, das o/p-Verhältnis betrug 0,64.

Beispiel 23

Das Verfahren des Beispiels 1 wurde wiederholt, man setzte jedoch 0,005 Gew.-Teile des Co-Katalysators der Formel

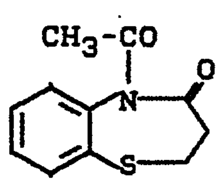

(5-Acetyl-2,3-dihydro-5H-benzo[b][1,4]thiazepin-4-on)

zu und erhitzte auf 50°C. Der Toluolgehalt betrug 2,3 Gew.-%, das o/p-Verhältnis 0,77.

Beispiel 24

Das Verfahren von Beispiel 1 wurde wiederholt, man setzte jedoch 0,004 Gew.-Teile des Co-Katalysators der Formel

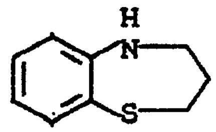

(2,3,4,5-Tetrahydro-benzo[b][1,4]thiazepin)

zu und erhitzte auf 45°C. Der Resttoluolgehalt betrug 3,9 Gew.-%, das o/p-Verhältnis 0,91.

Beispiel 25

Das Verfahren von Beispiel 1 wurde wiederholt, man setzte jedoch 0,0045 Gew.-Teile des Co-Katalysators der Formel

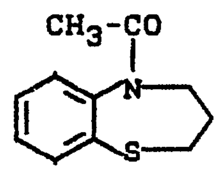

(5-Acetyl-2,3,4,5-tetrahydro-benzo[b][1,4]thiazepin)

zu und erhitzte auf 50°C. Der Toluolgehalt betrug 7,8 Gew.-%, das o/p-Verhältnis 1,35.

Beispiel 26

Das Verfahren von Beispiel 1 wurde wiederholt, man setzte jedoch 0,008 Gew.-Teile des Co-Katalysators der Formel

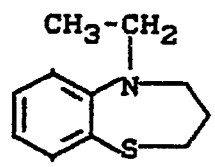

(5-Ethyl-2,3,4,5-tetrahydro-benzo[b][1,4]thiazepin)

zu und erhitzte auf 50°C. Der Toluolrestgehalt betrug 5,7 Gew.-%, das o/p-Verhältnis 0,93.

Beispiel 27

Das Verfahren von Beispiel 1 wurde wiederholt, man setzte jedoch 0,0045 Gew.-Teile des Co-Katalysators der Formel

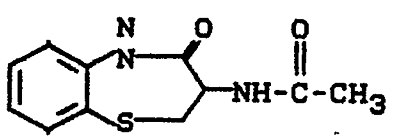

(3-Acetamino-2,3-dihydro-5H-benzo[b][1,4]thiazepin-4-on)

zu und erhitzte auf 50°C. Der Toluolgehalt betrug 5,6 Gew.-%, das o/p-Verhältnis 0,99.

Beispiel 28

Das Verfahren von Beispiel 1 wurde wiederholt, man legte jedoch 0,008 Gew.-Teile des Co-Katalysators der Formel

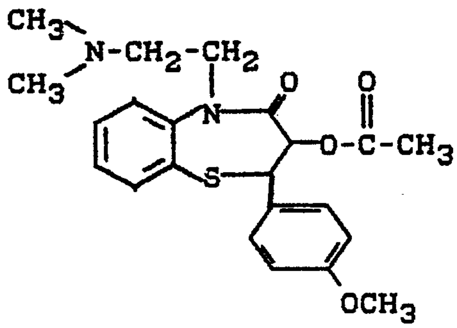

(5[(2-Dimethylamino) ethyl]-3-acetoxy-2-(4-methoxyphenyl)-5H-benzo[b][1,4] thiazepin-4-on)

zu und erhitzt auf 50°C. Der Toluolgehalt betrug 8,9 Gew.-%, das o/p-Verhältnis 1,34.

Beispiel 29

Das Verfahren von Beispiel 1 wurde wiederholt, man setzte jedoch 0,005 Gew.-Teile des Co-Katalysators der Formel

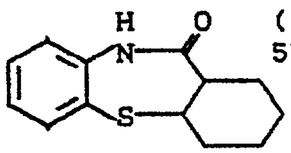

(2,3-Tetramethylen-2,3-dihydro-5H-benzo[b][1,4]thiazepin-4-on)

zu und erhitzte auf 50°C. Der Resttoluolgehalt betrug 4,1 Gew.-%, das o/p-Verhältnis 0,68.

Beispiel 30

Das Verfahren von Beispiel 1 wurde wiederholt. Man setzte jedoch 0,035 Gew.-Teile $FeCl_3$ und 0,050 Gew.-Teile des Co-Katalysators der Formel

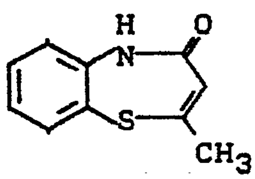

(2-Methyl-5H-benzo[b][1,4]thiazepin-4-on)

zu. Der Toluolrest betrug 5,2 Gew.-%, das o/p-Verhältnis 1,44.

Beispiel 31

Das Verfahren von Beispiel 1 wurde wiederholt. Man setzte jedoch 0,035 Gew.-Teile $FeCl_3$ and 0,013 Gew.-Teile des Co-Katalysators aus Beispiel 6 zu und erhitzte auf 50°C. Der Toluolgehalt betrug 3,5 Gew.-%, das o/p-Verhältnis 0,77.

Beispiel 32

Das Verfahren des Beispiels 1 wurde wiederholt. Man setzte jedoch 0,001 Gew.-Teile des Co-Katalysators des Beispiels 6 zu und erhitzte auf 50°C. Der Toluolgehalt betrug 5,3 Gew.-%, das o/p-Verhältnis 0,77.

Beispiel 33

Das Verfahren des Beispiels 1 wurde wiederholt. Man setzte jedoch 0,0055 Gew.-Teile einer Mischung von ca. 95 % des Co-Katalysators der Formel

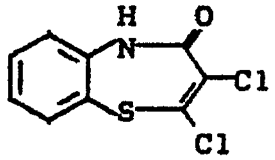

(2,3-Dichlor-5H-benzo[b][1,4]thiazepin-4-on)

und ca. 5 % des Co-Katalysators der Formel

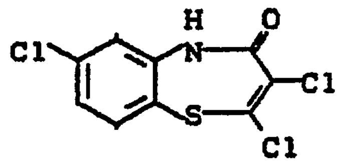

(2,3,7-Trichlor-5H-benzo[b][1,4]thiazepin-4-on)

zu und erhitzte auf 50°C. Der Toluolgehalt betrug 4,6 %, das o/p-Verhältnis 1,19.

Beispiel 34

Das Verfahren des Beispiels 1 wurde wiederholt, man setzte jedoch 0,006 Gew.-Teile des Co-Katalysators der Formel

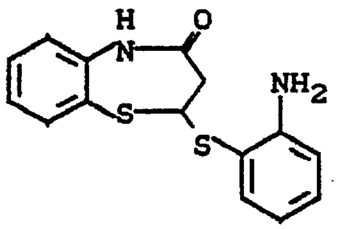

(2-(2-Aminophenylthio)-2,3-dihydro-5H-benzo[b][1,4]thiazepin-4-on)

zu und erhitzte auf 50°C. Der Resttoluolgehalt betrug 3,6 Gew.-%, das o/p-Verhältnis 1,08.

Beispiel 35

Das Verfahren des Beispiels 1 wurde wiederholt, man setzte jedoch 0,008 Gew.-Teile des Co-Katalysators der Formel

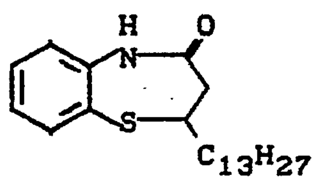

(2-n-Tridecyl-2,3-dihydro-5H-benzo[b][1,4]thiazepin-4-on)

zu. Der Toluolgehalt betrug 3,5 Gew.-%, das o/p-Verhältnis 0,71.

Beispiel 36

Das Verfahren des Beispiels 1 wurde wiederholt. Man setzte jedoch 0,0055 Gew.-Teile des Co-Katalysators der Formel

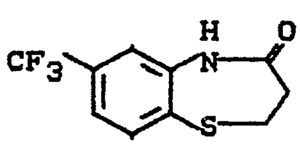

(7-Trifluormethyl-2,3-dihydro-5H-benzo-[b][1,4]thiazepin-4-on)

zu. Der Toluolgehalt betrug 4,4 Gew.-%, das o/p-Verhältnis 0,89.

Beispiel 37

Das Verfahren des Beispiels 1 wurde wiederholt. Man setzte jedoch 0,0045 Gew.-Teile des Co-Katalysators der Formel

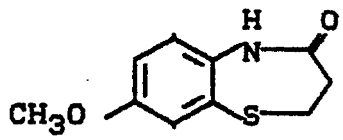

(8-Methoxy-2,3-dihydro-5H-benzo[b][1,4]thiazepin-4-on)

zu und erhitzte auf 45°C. Der Resttoluolgehalt betrug 4,0 Gew.-%, das o/p-Verhältnis 0,76.

Beispiel 38

Das Verfahren des Beispiels 1 wurde wiederholt. Man setzte jedoch 0,0055 Gew.-Teile des Co-Katalysators der Formel

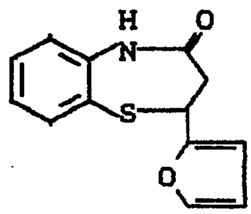

(2-(2-Furyl)-2,3-dihydro-5H-benzo[b][1,4]thiazepin-4-on)

zu und erhitzte auf 50°C. Der Resttoluolgehalt betrug 4,4, Gew.-%, das o/p-Verhältnis 1,10.

Beispiel 39

Das Verfahren des Beispiels 1 wurde wiederholt. Man setzte jedoch 0,0055 Gew.-Teile des Co-Katalysators der Formel

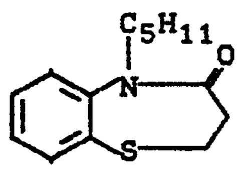

(5-n-Pentyl-2,3-dihydro-5H-benzo[b][1,4]thiazepin-4-on)

zu. Der Resttoluolgehalt betrug 3,4 Gew.-%, das o/p-Verhältnis 0,99.

Beispiel 40

Das Verfahren des Beispiels 1 wurde wiederholt. Man setzte jedoch 0,0047 Gew.-Teile des Co-Katalysators der Formel

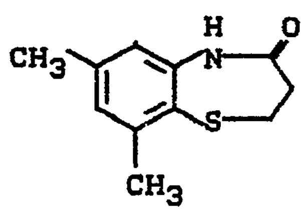

(7,9-Dimethyl-2,3-dihydro-5H-benzo[b][1,4]thiazepin-4-on)

zu und erhitzte auf 50°C. Der Resttoluolgehalt betrug 2,8 Gew.-%, das o/p-Verhältnis 0,71.

Beispiel 41

Das Verfahren des Beispiels 1 wurde wiederholt. Man setzte jedoch 0,0059 Gew.-Teile des Co-Katalysators der Formel

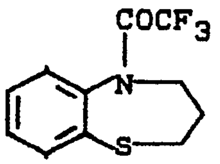

(5-Trifluoracetyl-2,3,4,5-tetrahydro-benzo[b][1,4] thiazepin)

zu und erhitzte auf 50°C. Der Resttoluolgehalt betrug 3,8 Gew.-%, das o/p-Verhältnis war 1,12.

Beispiel 42

Das Verfahren des Beispiels 1 wurde wiederholt. Man setzte jedoch 0,0069 Gew.-Teile des Co-Katalysators der Formel

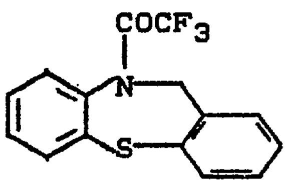

(5-Trifluoracetyl-5H-dibenzo[b,f][1,4]thiazepin)

zu und erhitzte auf 50°C. Der Resttoluolgehalt betrug 2,8 Gew.-%, das o/p-Verhältnis 0,97.

Beispiel 43

Das Verfahren des Beispiels 1 wurde wiederhol. Man setzte jedoch 0,0051 Gew.-Teile des Co-Katalysators der Formel

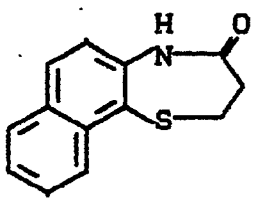

(2,3-Dihydro-5H[1,2]-naphthaleno[b][1,4] thiazepin-4-on)

zu. Der Resttoluolgehalt betrug 3,2 Gew.-%, das o/p-Verhältnis 0,76.

Beispiel 44

Das Verfahren von Beispiel 1 wurde wiederholt, man gab jedoch 0,0046 Gew.-Teile des Co-Katalysators der Formel

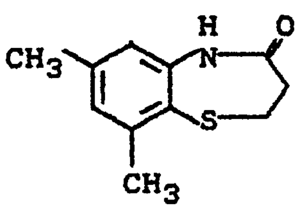

(7,9-Dimethyl-2,3-dihydro-5H-benzo[b][1,4]thiazepin-4-on)

zu. Das abgeführte Reaktionsgemisch enthielt 7,5 Gew.-% Toluol, das o/p-Verhältnis betrug 0,68.

Beispiel 45

Das Verfahren von Beispiel 1 wurde wiederholt, man gab jedoch 0,0053 Gew.-Teile des Co-Katalysators der Formel

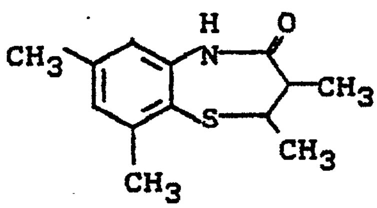

(2,3,7,9-Tetramethyl-2,3-hydro-5H-benzo[b][1,4]thiazepin-4-on)

zu. Das abgeführte Reaktionsgemisch enthielt 7,5 Gew.-% Toluol, das o/p-Verhältnis betrug 0,65.

Beispiel 46

Das Verfahren von Beispiel 20 wurde wiederholt, man gab jedoch 0,0057 Gew.-Teile des Co-Katalysators der Formel

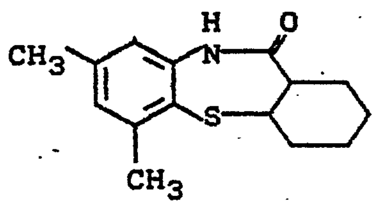

(7,9-Dimethyl-2,3-tetramethylen-2,3-dihydro-5H-benzo[b]-[1,4]thiazepin-4-on)

zu. Das abgeführte Reaktionsgemisch enthielt 7,0 Gew.-% Toluol, das o/p-Verhältnis betrug 0,64.

Beispiel 47

Das Verfahren von Beispiel 1 wurde wiederholt, man gab jedoch 0,0047 Gew.-Teile des Co-Katalysators der Formel

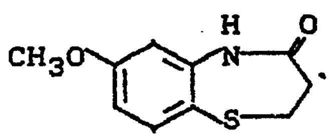

(7-Methoxy-2,3-dihydro-5H-benzo[b][1,4]thiazepin-4-on]

zu. Der Resttoluolgehalt betrug 3,6 Gew.-%, das o/p-Verhältnis war 0,70.

Beispiel 48

Das Verfahren von Beispiel 1 wurde wiederholt, man gab jedoch 0,0053 Gew.-Teile des Co-Katalysators der Formel

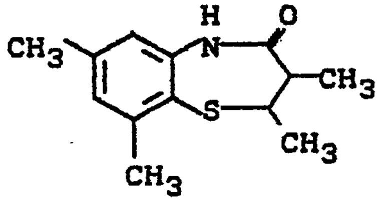

(2,3,7,9-Tetramethyl-2,3-dihydro-5H-benzo[b][1,4]-thiazepin-4-on)

zu. Der Resttoluolgehalt betrug 3,1 Gew.-%, das o/p-Verhältnis war 0,67.

Beispiel 49

Das Verfahren von Beispiel 1 wurde wiederholt, man gab jedoch 0,0057 Gew.-Teile des Co-Katalysators der Formel

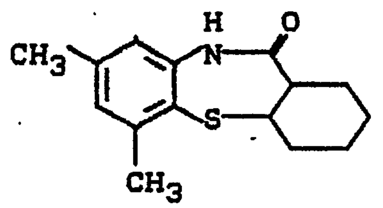

(7,9-Dimethyl-2,3-tetramethylen-2,3-dihydro-5H[b]-[1,4]thiazepin-4-on)

zu. Der Resttoluolgehalt betrug 3,4-Gew.-%, das o/p-Verhältnis betrug 0,64.

Beispiel 50 (Vergleichsbeispiel)

In 100 Gew.-Teilen Toluol wurden 0,7 Gew.-Teile $FeCl_3$ und 0,29 Gew.-Teile des nach der EP 0 173 222-Vorschrift hergestellten Phenoxathiinderivats gelöst. Man leitete bei 50°C ca. 94 Mol-% Chlor gasförmig unter Rühren ein. Der Restgehalt an Toluol betrug 7,9 %, das o/p-Verhältnis 0,88.

Beispiel 51 (Vergleichsbeispiel)

Das Verfahren des Beispiels 50 wurde wiederholt. In 100 Gew.-Teilen Toluol wurden 0, 0175 Gew.-Teile $FeCl_3$ und 0,008 Gew.-Teile des nach der EP 0 173 222-Vorschrift hergestellten Phenoxathiinderivats gelöst. Man leitete bei 50°C ca. 94 mol-% $Cl_2$ gasförmig unter Rühren ein. Der Restgehalt an Toluol betrug 6,4 Gew.-%, das o/p-Verhältnis 1,26.

Beispiel 52 (Vergleichsbeispiel)

Das Verfahren des Beispiels 50 wurde wiederholt. In 100 Gew.-Teilen Toluol wurden 0,0175 Gew.-Teile $FeCl_3$ und 0,0065 Gew.-Teile des in Beispiel 4 der US-P 4 031 147 genannten Co-Katalysators der Formel

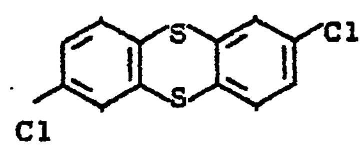

(2,7-Dichlorthianthren)

gelöst. Man erhitzte auf 50°C und leitete unter Rühren ca. 94 mol-% $Cl_2$ gasförmig ein. Der Resttoluolgehalt betrug 6,7 Gew.-%, das o/p-Verhältnis 1,55.

Beispiel 53 (Vergleichbeispiel)

Das Verfahren des Beispiels 50 wurde wiederholt. In 100 Gew.-Teilen Toluol wurden 0,0175 Gew.-Teile $FeCl_3$ und 0,006 Gew.-Teile des in Beispiel 1 der EP 0 126 669 genannten Co-Katalysators der Formel

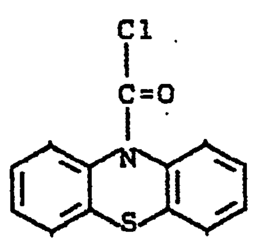

(N-Chlorcarbonylphenothiazin)

gelöst. Man erhitzte auf 50°C und leitete unter Rühren ca. 94 Mol-% Chlor gasförmig ein. Der Resttoluolgehalt betrug 5,6 Gew.-%, das o/p-Verhältnis 1,04.

## Patentansprüche

1. Verfahren zur Kernchlorierung von aromatischen Kohlenwasserstoffen der Formel

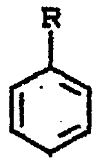

worin R einen Alkyl- oder Cycloalkylrest mit bis zu 12 C-Atomen bedeutet, in Gegenwart von Friedel-Crafts-Katalysatoren und in Gegenwart von Co-Katalysatoren in flüssiger Phase, dadurch gekennzeichnet, daß man als Co-Katalysatoren Thiazepine einsetzt.

2. Verfahren nach Anspruch 1, dadurch gekennzeichnet, daß man Thiazepine der Formeln

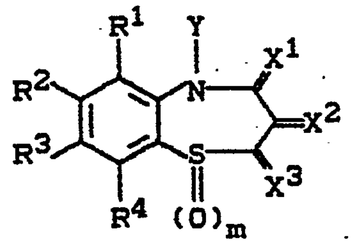

oder

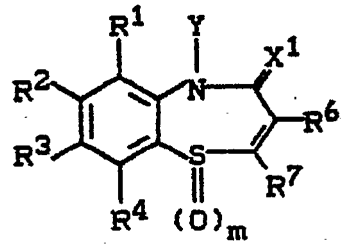

oder

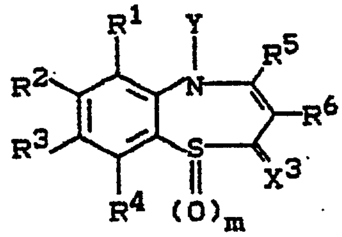

oder

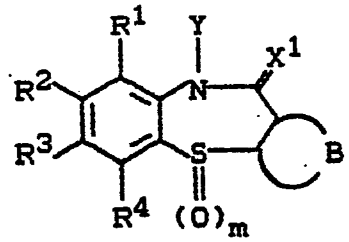

oder

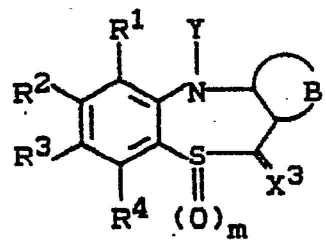

oder

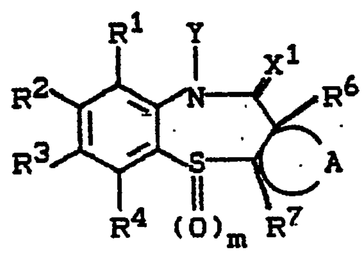

oder

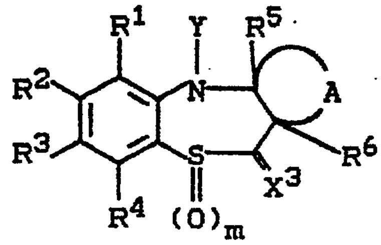

worin

$R^1$, $R^2$, $R^3$, $R^4$ gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxylamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen oder untereinander einen oder mehrere gesättigte oder ungesättigte, gegebenenfalls substituierte isocyclische oder heterocyclische Kohlenstoffringe mit bis zu 8 C-Atomen bilden,

Y Wasserstoff, gegebenenfalls substituiertes Alkyl, Aryl, Heteroaryl, Acyl, Thioacyl, Acyloxy, Arylamino oder Acylamino bedeutet,

$X^1$, $X^2$ oder $X^3$ folgende Gruppierungen bedeutet:

=O, =S, =N-Z, 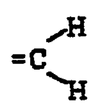 , 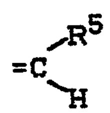 , 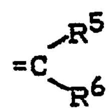

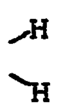 , 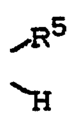 , 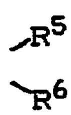 ,

$R^5$, $R^6$ und $R^7$ gleich oder verschieden sind und die Bedeutung von $R^1$ bis $R^4$ besitzen, mit der Ausnahme, daß sie untereinander keinen cyclischen Ring bilden,

Z die Bedeutung von Y hat mit der Ausnahme, daß Z nicht gleich H ist,

A die Anellierung eines gegebenenfalls substituierten gesättigten isocyclischen oder heterocyclischen Rings mit bis zu 8 C-Atomen bedeutet,

B die Anellierung eines gegebenenfalls substituierten ungesättigten isocyclischen oder heterocyclischen Rings mit bis zu 8 C-Atomen bedeutet und

m 0 oder 1 bedeutet,

einsetzt.

3. Verfahren nach Ansprüchen 1 und 2, dadurch gekennzeichnet, daß die Menge an eingesetztem Co-Katalysator 0,0001 bis 0,5 Gew.-%, bezogen auf den eingesetzten aromatischen Kohlenwasserstoff, beträgt.

4. Verfahren nach Ansprüchen 1 bis 3, dadurch gekennzeichnet, daß die Menge an eingesetztem Co-Katalysator 0,001 bis 0,1 Gew.-%, bezogen auf den eingesetzten aromatischen Kohlenwasserstoff, beträgt.

## Claims

1.Process for nucleus-chlorination of aromatic hydrocarbons of the formula

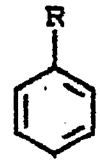

wherein R denotes an alkyl or cycloalkyl radical with up to 12 C atoms, in the presence of Friedel-Crafts catalysts and in the presence of co-catalysts in the liquid phase, characterized in that thiazepines are used as co-catalysts.

2. Process according to Claim 1, characterized in that thiazepines of the formulae

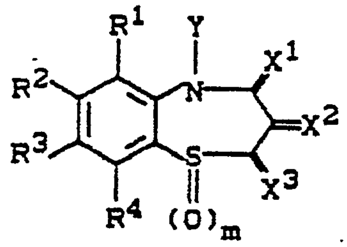

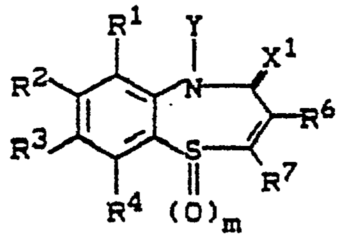

or

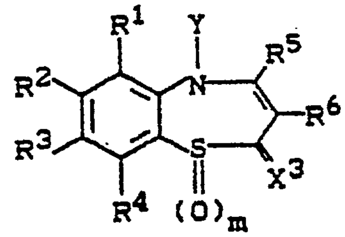

or

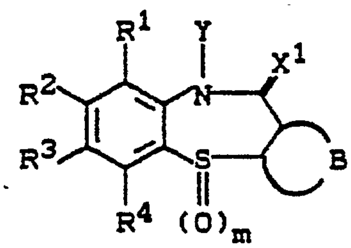

or

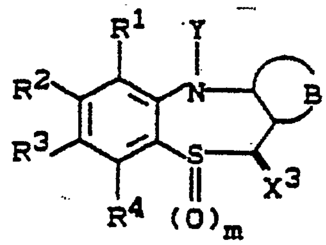

or

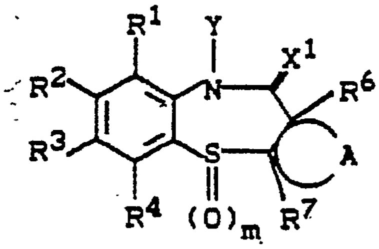

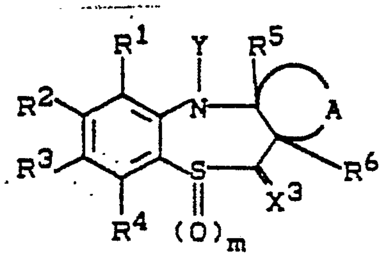

wherein

R1, $R^2$, $R^3$ and $R^4$ are identical or different and represent hydrogen, hydroxyl, amino, cyano, halogen, nitro, nitroso, sulphonyl, sulphoxyl, tosyl, mercapto, carboxyl, carboxyamide, carbalkoxy, dithiocarboxyl, thiocarboxylamide, dithiocarbalkoxy or optionally substituted alkyl, aryl, heteroaryl, alkoxy, aryloxy, heteroaryloxy, acyloxy, alkylthio, arylthio, heteroarylthio, acylthio, acyl, thioacyl or acylamino, or amongst themselves form one or more saturated or unsaturated optionally substituted isocyclic or heterocyclic carbon rings with up to 8 C atoms, Y denotes hydrogen or optionally substituted alkyl, aryl, heteroaryl, acyl, thioacyl, acyloxy, arylamino or acylamino,

$X^1$, $X^2$ or $X^3$ denotes the following groupings:

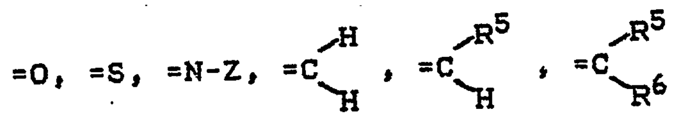

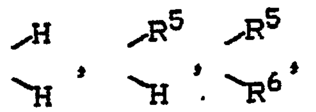

$R^5$, $R^6$ and $R^7$ are identical or different and have the meaning of $R^1$ to $R^4$, with the exception that they do not form a cyclic ring amongst themselves, Z has the meaning of Y, with the exception that Z is not H, A denotes the fusing-on of an optionally substituted saturated isocyclic or heterocyclic ring with up to 8 C atoms, B denotes the fusing-on of an optionally substituted unsaturatedisocyclic orheterocyclic ring with up to 8 C atoms and m denotes 0 or 1, are used.

3. Process according to Claims 1 and 2, characterized in that the amount of co-catalyst used is 0.0001 to 0.5% by weight, based on the aromatic hydrocarbon employed.

4. Process according to Claims 1 to 3, characterized in that the amount of co-catalyst used is 0.001 to 0.1% by weight, based on the aromatic hdyrocarbon employed.

**Revendications**

1. Procédé pour chlorer dans le noyau des hydrocarbures aromatiques de formule:

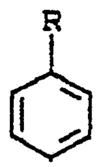

dans laquelle R représente un groupe alkyle ou cycloalkyle contenant jusqu'à 12 atomes de carbone, en présence de catalyseurs de Friedel-Crafts et en présence de catalyseurs auxiliaires en phase liquide, caractérisé en ce que l'on utilise en tant que catalyseurs auxiliaires des thiazépines.

2. Procédé selon la revendication 1, caractérisé en ce que l'on utilise des thiazépines de formule:

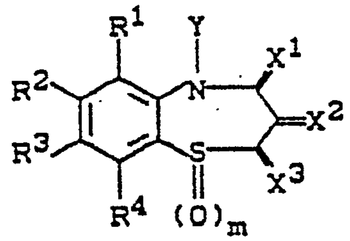

ou

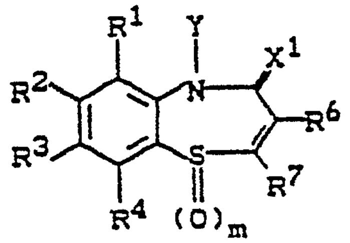

ou

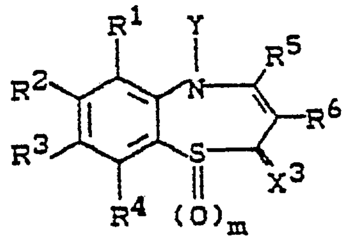

ou

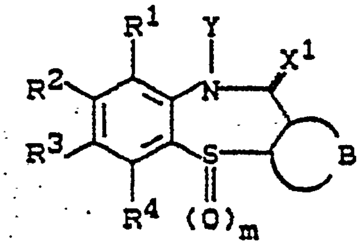

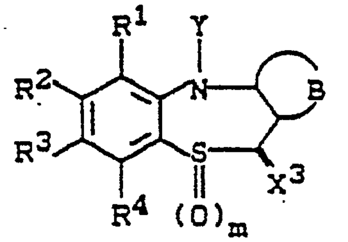

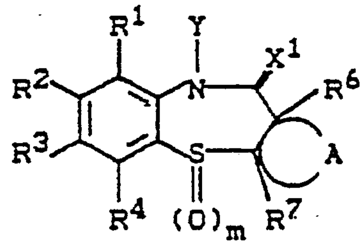

dans laquelle

$R^1$, $R^2$, $R^3$ et $R^4$, ayant des significations identiques ou différentes, représentent chacun l'hydrogène, un groupe hydroxy, amino, cyano, un halogène, un groupe nitro, nitroso, sulfonyle, sulfoxyle, toluènesulfonyle, mercapto, carboxyle, carboxamide, carbalcoxy, dithiocarboxyle, thiocarboxamide, dithiocarbalcoxy, alkyle, aryle, hétéroaryle, alcoxy, aryloxy, hétéroaryloxy, acyloxy, alkylthio, arylthio, hétéroarylthio, acylthio, acyle, thioacyle ou acylamino éventuellement substitué ou forment entre eux un ou plusieurs noyaux carbonés isocycliques ou hétérocycliques, saturés ou insaturés, éventuellement substitués, contenant jusqu'à 8 atomes de carbone, Y représente l'hydrogène, un groupe alkyle, aryle, hétéroaryle, acyle, thioacyle, acyloxy, arylamino ou acylamino éventuellement substitué,

$X^1$, $X^2$ ou $X^3$ représentent les groupements suivants:

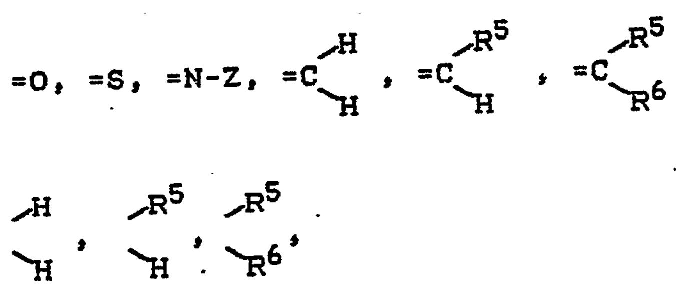

$R^5$, $R^6$ et $R^7$, ayant des significations identiques ou différentes, ont les mêmes significations que $R^1$, à $R^4$, sauf qu'ils ne peuvent former de noyaux cycliques entre eux, Z a les mêmes significations que Y, sauf qu'il ne peut représenter l'hydrogène, A indique la condensation d'un noyau isocyclique ou hétérocyclique saturé éventuellement substitué contenant jusqu'à 8 atomes de carbone, B indique la condensation d'un noyau isocyclique ou hétérocyclique insaturé éventuellement substitué contenant jusqu'à 8 atomes de carbone, et m est égal à 0 ou 1.

3. Procédé selon les revendications 1 et 2, caractérisé en ce que la quantité de catalyseur auxiliaire utilisée est de 0,0001 à 0,5% du poids de l'hydrocarbure aromatique mis en œuvre.

4. Procédé selon les revendications 1 à 3, caractérisé en ce que la quantité de catalyseur auxiliaire utilisée est de 0,001 à 0,1% du poids de l'hydrocarbure aromatique mis en œuvre.